# EUROPÄISCHE PATENTSCHRIFT

(11) **EP 2 688 641 B1**
(45) Veröffentlichungstag und Bekanntmachung des Hinweises auf die Patenterteilung: **11.02.2015**
(21) Anmeldenummer: 12720278.6
(22) Anmeldetag: 21.03.2012
(51) Int. Cl.: A61N 1/36, A61N 1/05, A61N 1/04, A61H 39/00, H05K 1/11, H05K 1/02, H05K 5/00

(54) **GERÄT ZUR PUNKTUAL-STIMULATION**
DEVICE FOR PUNCTUAL STIMULATION
APPAREIL DE STIMULATION PONCTUELLE

(30) Priorität: 23.03.2011 AT 1682011
(43) Veröffentlichungstag der Anmeldung: 29.01.2014
(73) Patentinhaber: Szeles, Josef Constantin, A-1190 Wien (AT)
(72) Erfinder: Szeles, Josef Constantin, A-1190 Wien (AT)
(74) Vertreter: Rentsch Partner AG
(86) Internationale Anmeldenummer: PCT/IB2012/000559
(87) Internationale Veröffentlichungsnummer: WO 2012/127306

(56) Entgegenhaltungen:
- WO-A1-01/35897
- WO-A1-2011/030210
- WO-A2-2009/154458
- DE-A1-102005 003 735
- US-A1- 2010 168 822
- SATOR-KATZENSCHLAGER SABINE M ET AL: "P-Stim auricular electroacupuncture stimulation device for pain relief", EXPERT REVIEW OF MEDICAL DEVICES, FUTURE DRUGS LTD., LONDON, GB, Bd. 4, Nr. 1, 1. Januar 2007 (2007-01-01), Seiten 23-32, XP009141474, ISSN: 1743-4440

## Beschreibung

Die Erfindung bezieht sich auf ein Gerät zur Punktual-Stimulation von im Bereich der Ohren liegenden Endigungen von zu Hirnstammkernen führenden Nerven, welches Gerät einen batteriegespeisten Behandlungsstromgenerator aufweist, der in einem im Ohrbereich zu tragenden Gehäuse angeordnet ist und der mit einer niederfrequenten Behandlungsstrom formenden Elektronikschaltung versehen ist, und welches Gerät weiter mindestens eine vom Behandlungsstromgenerator ausgehende flexible Leitung zur Verbindung mit einer an einer Nervenendigung zu positionierenden Elektrode und weiter eine gleichfalls an den Behandlungsstromgenerator angeschlossene Basiselektrode aufweist, über die der über die erstgenannte Elektrode führende Behandlungsstromkreis geschlossen wird.

WO 01/35897 A1 beschreibt ein Gerät gemäss dem Oberbegriff von Anspruch 1 und zeigt ein Punktual-Stimulations-Therapiegerät mit einem batteriebetriebenen Behandlungsstromgenerator, der in einem kleinen, am Körper zu tragenden Gehäuse angeordnet ist und über mindestens eine flexible Leitung eine daran angeschlossene Punktual-Stimulations-Elektrode speist. Der Behandlungsstromgenerator ist mit einem den Behandlungsstromverlauf steuernden programmierbaren Mikroprozessor bzw. Mikrocontroller versehen. Das Gehäuse des Gerätes kann annähernd die Form einer Kalotte haben. An der Basisfläche der Kalotte trägt das Gehäuse eine über diese Basisfläche hinausragende klebehaftend ausgebildete, flexible Flächenelektrodenscheibe, mit einem im Wesentlichen gleich breiten Rand.

US 2010/0168822 A1 zeigt ein Punktual-Stimulations-Therapiegerät mit einem batteriebetriebenen Behandlungsstromgenerator in einer Tragstruktur, die im Bereich des Halses oder des Oberarms des Patienten angeordnet ist, über mit einer flexiblen Leitung eine daran angeschlossene Punktual-Stimulations-Elektrode speist. Die genannte Elektrode hat die Form eines Ohrstöpsels. In einer anderen Ausführungsform hat das PunktualStimulations-Therapiegerät ein am Ohr zu tragendes bügelförmiges Gehäuse.

DE 102005003735 A1 zeigt eine Vorrichtung zur transkutanen Stimulation eines Nervs des menschlichen Körpers, die mindestens eine Stimulationselektrode und mindestens eine Referenzelektrode zur transkutanen Nervenstimulation aufweist, wobei die mindestens eine Stimulationselektrode und die mindestens eine Referenzelektrode mit einer Steuereinheit in Verbindung stehen und von dieser mit einem elektrischen Strom beaufschlagt werden können. Diese Elektroden sind in oder an einem Gehäuse angeordnet, dass zur Anbringung am menschlichen Ohr geeignet ist.

WO 2009/154458 A2 zeigt ein elektronisches Stimulationsgerät, dass hinter dem Ohr zu tragen ist, und einem in Bereich der Nase anzubringenden Sensor, der die Atmungsaktivität misst, und die Messdaten drahtlos an das Stimulationsgerät überträgt. Bei sich entwickelndem Atmungsstillstand werden elektrische Impulse an Stimulationspunkte am Ohr abgegeben.

Es ist für eine gute Wirkung einer über längere Zeiträume mit einem Gerät vorgenannter Art durchzuführenden Stimulationsbehandlung ein guter Sitz eines solchen Gerätes am Körper eines zu behandelnden Patienten bedeutsam. Es ist nun ein Ziel der Erfindung eine Ausbildung eines Gerätes vorgenannter Art zu schaffen, welche beim Anbringen des Gerätes an einem Patienten mit einer einfachen Handhabung einen guten Sitz erzielen lässt und die gegenüber den Sitz störenden Einflüssen, wie z.B. körperpflegende Massnahmen im Nassbereich, einigermassen widerstandsfähig ist. Es sollen dabei möglichst auch keine grob in Erscheinung tretenden Halteelemente erforderlich sein und es soll die zu schaffende Ausbildung mit geringem Aufwand realisierbar sein.

Die Erfindung betrifft ein Gerät gemäss Anspruch 1. Die abhängigen Ansprüche betreffen bevorzugte Ausführungsformen.

Das erfindungsgemäss ausgebildete Gerät ist mindestens mit einem aussenliegenden Teil dieses Gerätes, an die Form der zu seiner Positionierung vorgesehenen Körperstelle eines Patienten durch mechanisch nachgiebige Struktur anpassbar Durch diese Ausbildung kann der vorstehend angeführten Zielsetzung gut entsprochen werden. Beim Anbringen des Gerätes an einem Patienten kann durch die mechanisch nachgiebige Struktur auf einfache Weise ein Anliegen des Gerätes an der vorgesehenen Körperstelle erzielt werden; das Anliegen am Körper wirkt einem unerwünschten Abheben vom Körper und einem unerwünschten Verschieben am Körper unter dem Einfluss äusserer Kräfte, die bei der Körperpflege auftreten können, entgegen. Für viele Anwendungen ist es günstig eine flä-chenhafte Basiselektrode vorzusehen, welche mechanisch nachgiebig ausgebildet ist und zum Auflegen auf eine im Ohrbereich eines Patienten befindliche Hautstelle vorgesehen ist. Die Basiselektrode bildet dabei einen außen liegenden Teil des Gerätes und kann auch allein einen mechanisch nachgiebigen Teil realisieren. Ein gutes Anliegen der Basiselektrode ergibt über längere Zeit gleichbleibende Verhältnisse im Stromkreis des Behandlungsstromes. Eine baulich einfache Ausführungsform des erfindungsgemäßen Gerätes, welche ein gutes Anschmiegen der Basiselektrode an die vorgesehene Körperstelle erzielen lässt, ist dadurch gekennzeichnet, dass die im Behandlungsstromkreis liegende Basiselektrode des Gerätes flächenhaft flexibel ausgebildet ist und fahnenartig beweglich am Gehäuse des Gerätes angeordnet ist. Hierbei ist es im Sinn eines guten Anschmiegens und für eine gute elektrische Kontaktierung weiter vorteilhaft, wenn man vorsieht, dass die Basiselektrode elektrisch leitfähige Streifen oder Fäden aufweist, welche vorzugsweise gitterartig oder netzartig verlaufen.

Für den Sitz des Gehäuses des Gerätes an der für das Anordnen des Gerätes vorgesehenen Körperstelle ist es günstig, wenn man vorsieht, dass die Basiselektrode flächenhaft und flexibel ausgebildet ist und die Wand des Gehäuses des Gerätes mindestens an jener Seite, an der die flächenhafte Basiselektrode angeordnet ist, flexibel ausgebildet ist. Hierbei ist es weiter vorteilhaft, wenn eine flächenhafte Basiselektrode an der flexibel ausgebildeten Wand des Gehäuses anliegend angeordnet ist. Die Basiselektrode kann dabei mit der flexibel ausgebildeten Wand des Gehäuses des Gerätes flächig verbunden sein oder lose an dieser Wand positioniert sein.

Eine weitere vorteilhafte Ausbildung des erfindungsgemäßen Gerätes ist dadurch gekennzeichnet, dass die Basiselektrode eine Nadelelektrode ist, welche über eine flexible Leitung an den Behandlungsstromgenerator angeschlossen ist. Es kann auf diese Weise die Anschmiegung des mindestens zum Teil mechanisch nachgiebig ausgebildeten Gehäuses des Gerätes an eine Körperstelle eines Patienten vorgenommen werden, ohne dass es einer besonderen Rücksichtnahme auf die Wahl einer bestimmten Körperstelle für die Stromeinleitung über die Basiselektrode bedarf und es kann eine solche Körperstelle, an der die Basiselektrode vorgesehen werden soll, unabhängig vom Ort der Anschmiegung des Gerätegehäuses an den Körper gewählt werden.

Beim Anbringen des Gerätes am Körper eines Patienten kann vorteilhaft ein sich verfestigendes reizfreies bzw. reizarmes Gel eingesetzt werden, welches allfällig nach dem Ansetzen des Gerätes an den Körper zwischen diesem und der Wand des Gerätes verbliebene Spalte füllt und die Auflagestelle der Basiselektrode gegen das Eindringen von Fremdsubstanzen, insbesondere Wasser, abschließt. Eine vorteilhafte Variante ist dadurch gekennzeichnet, dass mindestens auf einem Teil der Aussenseite des Gehäuses des Gerätes eine beim Aufbringen bildsame und danach sich verfestigende Masse vorgesehen ist. Günstige Ergebnisse können dabei mit einer Silikonmasse erzielt werden.

Für ein exaktes Einhalten der für das Anbringen des Gerätes vorgesehenen Stelle ist es vorteilhaft, wenn das Gehäuse des Gerätes mit einem die Anbringung im Ohrbereich unterstützenden Positionierungsglied versehen ist. Hierfür ist eine vorteilhafte Ausführungsform dadurch gekennzeichnet, dass als Positionierungsglied eine den Rand des Ohres eines Patienten übergreifende Klappe oder Klemme vorgesehen ist. Eine andere günstige Ausführungsform ist dadurch gekennzeichnet, dass als Positionierungsglied am Gehäuse des Gerätes ein um den Rand des Ohres eines Patienten biegbarer plastisch verformbarer Haltestreifen vorgesehen ist. Solche Positionierungsglieder können auch als Basiselektrode wirksam sein.

Hinsichtlich des Assemblings der Bauelemente der zur Erzeugung des Behandlungsstromes dienenden Elektronikschaltung und der Unterbringung dieser Elektronikschaltung im Gehäuse des Gerätes, insbesondere wenn es sich um ein Gehäuse mit flexibler Wand handelt, ist die den Behandlungsstromgenerator bildende Elektronikschaltung auf einer flexiblen Folienplatine gebildet. Dabei ergibt sich eine weiter vorteilhafte Vereinfachung des Aufbaues, die auch eine erhöhte Flexibilität des Gehäuses erzielen lässt, wenn man vorsieht, dass die flexible Folienplatine mindestens einseitig elektrisch isolierend überdeckt ist und eine Wand des Gehäuses des Gerätes bildet. Baulich ist es dabei weiter günstig, wenn die Folienplatine auch eine Speisebatterie trägt. Es kann dabei mit geringem Platzbedarf das Auslangen gefunden werden und eine gute Flexibilität sowie ein bei gegebenen Gehäuseabmessungen großer Energieinhalt erzielt werden, wenn man vorsieht, dass die Speisebatterie ihrerseits einen aus flexiblen Folien gebildeten Mantel aufweist.

In dem erfindungsgemäßen Gerätes, ist vorgesehen, dass die flexible Folienplatine einen aus dem Gehäuse des Gerätes hinausragenden Abschnitt aufweist, auf dem sich mindestens eine an einen Ausgang des Behandlungsstromgenerators angeschlossene Leiterbahn befindet, die in einem durch seitliche Schnitte begrenzten Folienstreifen verläuft und zusammen mit diesem Folienstreifen eine von dem aus dem Gehäuse herausragenden Abschnitt der Folienplatine abziehbare flexible Leitung zur Verbindung mit einer in einen Stimulationspunkt einstechbaren Elektrode oder einer Sensorelektrode oder einer Basiselektrode bildet. Man kann auf diese Weise mit geringem Fertigungsaufwand zur Verbindung mit außen liegenden Elektroden dienende flexible Leitungen bilden, welche keiner besonderen Maßnahme zum Anschluss an die Elektronikschaltung bedürfen und in einem Arbeitsgang mit den auf der Platine zu bildenden internen Leiterbahnen der Elektronikschaltung gebildet werden können, die weiter auch eine gute mechanische Festigkeit bei gleichzeitig guter Flexibilität aufweisen und die auch leicht gehandhabt werden können und beim Verpacken des Gerätes keiner besonderen Sorgfalt bedürfen. Es ist dabei auch vorteilhaft, wenn der mindestens eine Folienstreifen, der eine Leiterbahn beinhaltet, durch in der Fläche der Folienplatine zick-zack-förmig verlaufende Schnitte begrenzt ist oder wenn der mindestens eine Folienstreifen der eine Leiterbahn beinhaltet, durch in der Fläche der Folienplatine spiralartig verlaufende Schnitte begrenzt ist. Dies ermöglicht es auf einem verhältnismäßig kleinen, aus dem Gehäuse des Gerätes herausragenden Abschnitt der Folienplatine abziehbare Leitungen größerer Länge zu bilden. Sieht man dabei ergänzend vor, dass einzelne Abschnitte des zick-zack-förmigen oder spiralartig geformten Folienstreifens untereinander und mit dem neben dem Folienstreifen verbliebenen Bereich der Folienplatine über durchtrennbare Stege verbunden sind, kann man die vom genannten Abschnitt der Folienplatine wegführenden Leitungen in der jeweils benötigten Länge abziehen, wobei der jeweils verbleibende Teil der Leitungslänge am genannten Abschnitt der Folienplatine fixiert bleibt, da von den genannten Stegen beim Abziehen der Leitungen nur jene Stege aufgetrennt werden, die die jeweils benötigte Leitungslänge halten.

Eine beispielhafte Form des erfindungsgemäßen Gerätes, welche sowohl hinsichtlich der Fertigung als auch hinsichtlich der Manipulation bei der Anbringung eines solchen Gerätes am Körper eines Patienten und auch eines dabei erzielbaren guten Sitzes und weitgehend sattem Anliegen am Körper Vorteile bietet, ist derart gestaltet dass das Gehäuse des Gerätes einen gitterartig oder netzartig ausgebildeten Korb aufweist, in dem mindestens ein Teil der elektrisch aktiven Bauelemente des Gerätes, insbesondere der Behandlungsstromgenerator, angeordnet ist und der die Basis für eine umschließende Umhüllung bildet, die aus einer zum Aufbringen auf den Korb bildsamen, danach aber sich verfestigenden Masse besteht. Der Korb weist durch die gitterartige oder netzartige Struktur eine gewisse Flexibilität auf und kann beim Anlegen des Gerätes oder bei einem manuellen Vorformen vor dem Anlegen annähernd der Form der Körperstelle, an der das Gerät platziert werden soll, angepasst werden und es werden die verbleibenden Formunterschiede zwischen dem Korb und der Körperoberfläche durch die bildsame Masse beim Anlegen des Gerätes gefüllt. Nach dem Anlegen des Gerätes verfestigt sich die bildsame Masse, wobei durch das Haften dieser Masse am Körper ein guter Sitz des Gerätes am Körper und ein Schutz der elektrisch aktiven Bauelemente vor Wasser und anderen Substanzen, die bei der Körperpflege verwendet werden, erhalten werden kann. Es ist günstig, wenn die bildsame Masse den mit den elektrisch aktiven Bauelementen versehenen Korb allseitig umgibt. Es ist aber auch möglich, dass der mit den elektrisch aktiven Bauelementen versehene Korb von der bildsamen Masse, die ringsum an der Körperoberfläche dicht anliegt, kappenartig überdeckt wird. Als bildsame Masse kann vorteilhaft eine sich verfestigende Silikonmasse vorgesehen werden. Die bildsame Masse findet durch die gitterartige oder netzartige Struktur des Korbes ohne besondere zusätzliche Maßnahmen einen guten Halt am Korb, wobei die genannte Struktur auch durch eine entsprechende Perforation einer Folie, aus der der Korb gebildet wird, erhalten werden kann. Es ist meist vorteilhaft, wenn der Korb elektrisch isolierend ausgebildet ist. Dies kann auch durch eine elektrisch isolierende Beschichtung bei einem aus elektrisch leitfähigem Material hergestellten Korb erzielt werden. Gegebenenfalls kann man auch ein elektrisch aktives Bauelement des Gerätes, welches mit den im Korb angeordneten Bauelementen zusammenarbeitet, wie z.B. eine zur Energieversorgung vorgesehene Speisebatterie, an der Außenseite des Korbes anordnen. Um die Manipulation beim Anbringen des Gerätes am Ohr möglichst zu vereinfachen ist es vorteilhaft am Korb ein Positionierungsglied anzuordnen. Mit einem solchen Positionierungsglied kann das Gerät zunächst platziert werden, worauf der Korb mit dem die Umhüllung bildenden Material umgeben werden kann, die das Gerät am Körper eines Patienten hält und vor nachteiligen, von außen kommenden Einflüssen schützt. Der Korb selbst kann allseitig geschlossen ausgebildet sein, wobei zu einer oder mehreren Elektroden führende Leitungen durch in der Korbstruktur vorliegende Öffnungen hindurchtreten, oder auch an mindestens einer Seite offen sein und z.B. die Form eines flexiblen Rohres aufweisen. Der Korb kann im Zuge der Herstellung des Gerätes in Art eines zumindest einseitig offenen Gefäßes vorgefertigt werden, in das danach elektrisch aktive Bauelemente eingefügt werden. Eine alternative Möglichkeit ist, elektrisch aktive Bauelemente auf einem Stück eines gitterartigen oder netzartigen Materials anzuordnen und dieses Material danach zu einem Korb zu formen.

Wenn das erfindungsgemäß ausgebildete Gerät mit einem die Anbringung dieses Gerätes im Ohrbereich eines Patienten unterstützenden Positionierungsglied in Form einer Klappe oder Klemme oder eines Haltestreifens versehen ist, ist es weiter günstig, wenn dieses Positionierungsglied eine Ausfütterung aus einem weichen, plastisch verformbaren Material aufweist. Eine solche Ausfütterung kann sich beim Anlegen des Positionierungsgliedes an das Ohr eines Patienten der Form der Anlegestelle anpassen und ergibt eine gute Haltewirkung ohne einen nachteilig empfundenen Druck auszuüben.

Das Gerät kann beispielsweise mit einem oder mehreren Sensoren versehen sein, die Körperfunktionen eines Patienten erfassen, z.B. einen Pulssensor, wobei solche Sensoren am Gehäuse des Gerätes angeordnet oder über Leitungen angeschlossen sein können und Signale zur Beeinflussung des Behandlungsstromes liefern können.

Die Erfindung wird nun an Hand von Ausführungsbeispielen unter Bezugnahme auf die Zeichnung, in der Ausführungsbeispiele schematisch dargestellt sind, weiter erläutert.

In der Zeichnung zeigt:
Fig. 1 ein erstes Ausführungsbeispiel eines erfindungsgemäßen Gerätes teilweise aufgeschnitten in einer Ansicht;
Fig. 2 ein Positionierungsglied eines erfindungsgemäßen Gerätes;
Fig. 3 ein anderes Ausführungsbeispiel eines erfindungsgemäßen Gerätes in einer Ansicht;
Fig. 4 ein Beispiel einer Basiselektrode eines erfindungsgemäßen Gerätes;
Fig. 5 ein weiteres Ausführungsbeispiel eines erfindungsgemäßen Gerätes in einer Ansicht;
Fig. 6 ein Ausführungsbeispiel eines erfindungsgemäßen Gerätes mit integrierten flexiblen Elektrodenzuleitungen;
Fig. 7 in größerem Maßstab eine Teilansicht eines erfindungsgemäßen Gerätes mit in einem Folienstreifen integrierten Elektrodenzuleitungen.
Fig. 8 ein Beispiel eines Gerätes bei dem das Gehäuse einen Korb aufweist, in einer schematisierten aufgebrochenen Ansicht;
Fig. 9 schematisiert die Platzierung eines solchen Gerätes im Ohrbereich;
Fig. 10 eine Variante der beim Beispiel nach Fig. 8 vorliegenden Korbstruktur; und
Fig. 11 eine andere Variante der beim Beispiel nach Fig. 8 vorliegenden Korbstruktur.
Fig. 12 zeigt in einer schematisierten Schrägansicht einen Teil eines Korbes, der zur Bildung des Gehäuses eines beispielhaften Gerätes vorgesehen ist, wobei in den Korb bereits zur Erzeugung des Behandlungsstromes dienende aktive Bauelemente eingefügt sind, und
Fig. 13 im schematisierten Schnitt ein Gerät, das aus der Anordnung gemäß Fig. 12 durch Hinzufügung einer umschließenden Umhüllung entstanden ist.
Fig. 14 zeigt in einem Schnitt ein mit einer Ausfütterung versehenes Positionierungsglied eines erfindungsgemäß ausbildeten Gerätes.

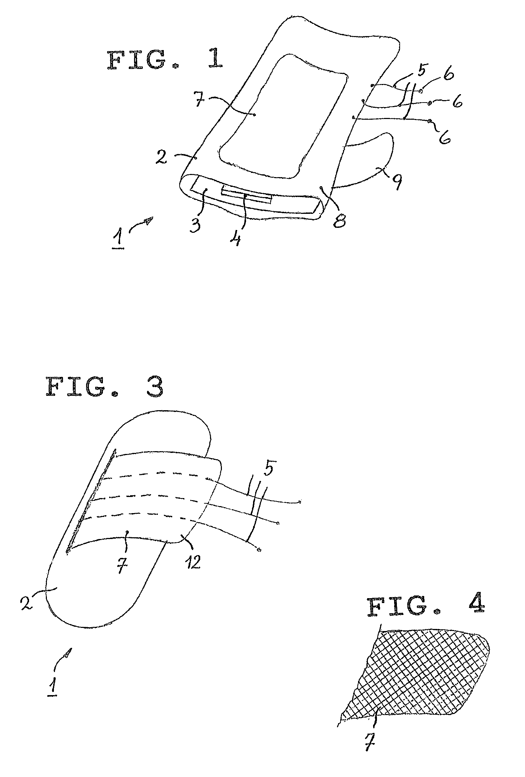

Das in Fig. 1 dargestellte Ausführungsbeispiel eines erfindungsgemäßen Gerätes 1 weist ein im Ohrbereich eines Patienten zu tragendes Gehäuse 2 auf, in dem ein auf einer Platine aufgebauter Behandlungsstromgenerator 3 und eine für dessen Betrieb dienende Speisebatterie 4 angeordnet ist. An den Behandlungsstromgenerator sind einerseits flexible Leitungen 5 und andererseits eine flächenhafte Basiselektrode 7 angeschlossen. An den flexiblen Leitungen 5 sind Anschlussstellen 6 für nicht näher dargestellte Elektroden vorgesehen, welche zur Stimulation von im Bereich der Ohren eines Patienten liegenden Endigungen von zu Hirnstammkernen führenden Nerven zu positionieren sind. Die flächenhaft ausgebildete Basiselektrode 7 liegt auf der Außenseite der Wand 8 des Gehäuses 2 auf und kann in Form einer dünnen Metallschicht realisiert sein, die z.B. die Form einer dünnen Folie oder eines Metallniederschlages haben kann. Auch andere, elektrisch leitende flächenhafte Materialien, welche flexibel sind, wie z.B. elektrisch leitfähig ausgerüstete Gewebe oder Vliese, kommen in Betracht. Über die Basiselektrode 7 wird der über die Leitungen 5 und über die nicht näher dargestellten, an den Nervenendigungen positionierten, Elektroden führende Behandlungsstromkreis geschlossen.

Das Gehäuse 2 und insbesondere dessen die Basiselektrode 7 tragende Wand 8, und damit auch die Basiselektrode 7 selbst, ist flexibel ausgebildet und durch seine mechanisch nachgiebige Struktur durch einfaches Andrücken an die Form der zur Positionierung der Basiselektrode 7 vorgesehenen Körperstelle eines Patienten anpassbar. Es ist dabei in der Regel auch günstig, wenn die Platine, auf der der Behandlungsstromgenerator 3 aufgebaut ist, flexibel ist.

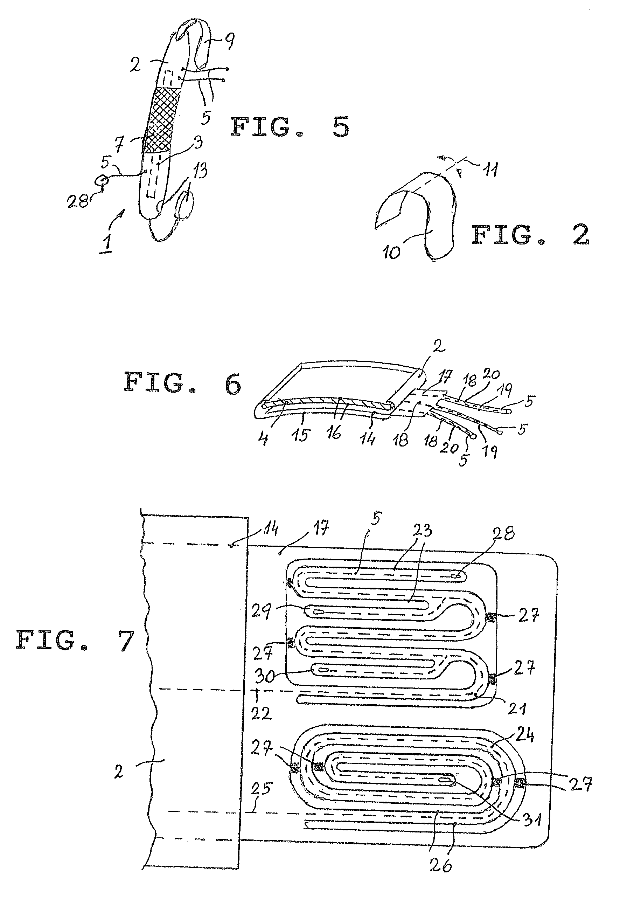

Das Gehäuse 2 des in Fig. 1 dargestellten Gerätes 1 ist mit einem dessen Anbringung im Ohrbereich eines Patienten unterstützenden Positionierungsglied in Form eines um den Rand des Ohres eines Patienten biegbaren, plastisch verformbaren Haltestreifen 9 versehen. Man kann dabei ergänzend zur Positionierung mit dem Haltestreifen 9 das Gerät mit einem sich verfestigenden Gel fixieren und auf diese Weise das Gerät gegen ein Abheben unter dem Einfluss äußerer Kräfte, die z.B. durch körperpflegende oder sportliche Aktivitäten entstehen können, schützen und auch einen unerwünschten Zutritt von Flüssigkeit zur Basiselektrode verhindern. Als Positionierungsglied kann auch, anstelle eines wie in Fig. 1 dargestellten Haltestreifens 9, eine den Rand des Ohres eines Patienten übergreifende Klappe oder Klemme vorgesehen werden. Ein Beispiel einer solchen Klappe oder Klemme 10 ist in Fig. 2 dargestellt und kann entweder einstückig in sich elastisch federnd oder aus zwei scharnierend miteinander verbundenen Teilen ausgeführt sein, wie durch eine strichlierte Achse 11 angedeutet ist.

Bei dem in Fig. 3 dargestellten Ausführungsbeispiel eines erfindungsgemäß ausgebildeten Gerätes 1 ist die Basiselektrode 7 fahnenartig beweglich am Gehäuse 2 angeordnet und flexibel ausgebildet. Die Basiselektrode 7 ist vorteilhaft so bemessen, dass ihr freies Ende 12 um den Rand des Ohres biegbar ist und die Basiselektrode damit die Positionierung des Gerätes erleichtern kann. Der Andruck der Basiselektrode an die Haut eines Patienten kann durch das Gehäuse 2 allein oder unter Mithilfe eines Gels erzielt werden. Das Gehäuse 2 selbst ist vorteilhaft flexibel ausgebildet, kann aber auch starr sein. Die zur Zufuhr des Behandlungsstromes an Stimulationselektroden vorgesehenen flexiblen Leitungen 5 können, wie in Fig. 3 dargestellt, an der flexiblen Basiselektrode 7 geführt sein, oder, ähnlich wie in Fig. 1 dargestellt, direkt vom Gehäuse 2 ausgehen. Die flexible Basiselektrode 7 kann in Form einer flexiblen Folie oder, wie in Fig. 4 dargestellt, in Form eines Gewebes oder Netzes, das elektrisch leitfähig ist, ausgeführt sein, wobei die elektrische Leitfähigkeit durch alle Fäden dieser Struktur oder durch ergänzend eingefügte leitende Fäden realisiert werden kann. Ein Gewebezuschnitt, dessen Fäden wie in Fig. 4 dargestellt schräg verlaufen, lässt eine besonders gute Schmiegsamkeit erzielen.

Das in Fig. 5 dargestellte Ausführungsbeispiel eines erfindungsgemäß ausgebildeten Gerätes 1 weist ein flexibles Gehäuse 2 auf, an dessen Wand eine flexible Basiselektrode 7 angeordnet ist. Von dem im Gehäuse 2 angeordneten Behandlungsstromgenerator 3 gehen flexible Leitungen 5 zur Zufuhr des Behandlungsstromes an Stimulationselektroden aus.

Weiter ist am Gehäuse 2 ein in Form eines Haltestreifens 9 oder in Form einer wie in Fig. 2 dargestellten Klappe oder Klemme 10 ausgebildetes Positionierungsglied vorgesehen.

Bei elektrisch leitfähiger Ausbildung eines in Form eines Haltestreifens 9 oder einer Klappe oder Klemme 10 ausgebildeten Positionierungsgliedes kann dieses auch in Ergänzung zur flexiblen Elektrode 7 oder anstelle derselben als Basiselektrode dienen und es kann auch in Ergänzung zur flexiblen Elektrode 7, oder anstelle derselben, eine über eine flexible Leitung 5 angeschlossene Nadelelektrode 28 als Basiselektrode vorgesehen sein.

Zur Einflussnahme auf die Steuerung der Stimulation weist das in Fig. 5 dargestellte Gerät weiter einen Körperfunktionen eines Patienten erfassenden Sensor, z.B. einen am Ohrläppchen zu positionierenden Pulssensor 13, auf.

Bei dem in Fig. 6 dargestellten Ausführungsbeispiel ist die den Behandlungsstromgenerator bildende Elektronikschaltung auf einer flexiblen Folienplatine 14 gebildet. Diese Folienplatine 14 ist auf einer Seite mit einer isolierenden Folie 15 überdeckt und bildet zusammen mit dieser eine Wand des Gehäuses 2 des Gerätes. An der der Folie 15 gegenüberliegenden Seite der Folienplatine 14 ist eine Speisebatterie 4 angeordnet, welche ihrerseits einen aus flexiblen Folien gebildeten Mantel 16 aufweist, der gleichfalls eine Wand des Gehäuses bildet.

Die Folienplatine weist einen aus dem Gehäuse 2 hinausragenden Abschnitt 17 auf, auf dem sich mehrere Leiterbahnen 18 befinden, die an einen oder mehrere Ausgänge des Behandlungsstromgenerators angeschlossen sind und in durch seitliche Schnitte 19 begrenzten Folienstreifen 20 verlaufen und flexible Leitungen 5 zur Verbindung mit Stimulationselektroden und/oder Sensorelektroden bilden.

Eine im Behandlungsstromkreis vorzusehende Basiselektrode kann flächenhaft ausgebildet und z.B. auf der Außenseite der Folie 15 oder auf der Außenseite des Mantels 16 aufliegend oder auch, wie in Fig. 3 dargestellt, fahnenartig beweglich am Gehäuse 2 angeordnet sein. Anstelle einer solchen flächenhaft ausgebildeten Basiselektrode oder in Ergänzung zu einer solchen flächenhaften Basiselektrode kann auch eine als Nadelelektrode ausgebildete Basiselektrode vorgesehen sein, welche über eine flexible Leitung, vorzugsweise eine auf dem Abschnitt 17 der Folienplatine 14 gebildete Leitung 5, an den auf der Platine 14 gebildeten Behandlungsstromgenerator angeschlossen ist.

Fig. 7 zeigt einen Teil eines erfindungsgemäß ausgebildeten Gerätes, bei dem die den Behandlungsstromgenerator bildende Elektronikschaltung auf einer flexiblen Folienplatine 14 gebildet ist, die einen aus dem Gehäuse 2 des Gerätes hinausragenden Abschnitt 17 besitzt, in dem flexible Leitungen 5 zur Verbindung mit Stimulationselektroden oder Sensorelektroden oder auch zur Verbindung mit einer als Basiselektrode dienenden Nadelelektrode gebildet sind. Es ist dabei ein Folienstreifen 21 dargestellt, der Leiterbahnen 22 beinhaltet und durch in der Fläche der Folienplatine 14 zick-zack-förmig verlaufende Schnitte 23 begrenzt ist. Weiter ist ein Folienstreifen 24 dargestellt, der eine Leiterbahn 25 beinhaltet und durch in der Fläche der Folienplatine 14 spiralartig verlaufende Schnitte 26 begrenzt ist. Einzelne Abschnitte der Folienstreifen 21 und 24 sind untereinander und mit dem neben den Folienstreifen verbliebenen Bereichen der Folienplatine 14 über durchtrennbare Stege 27 verbunden. Beim Abziehen der Folienstreifen 21 und 24 von deren Enden 28, 29, 30, 31 aus werden die Stege 27 nacheinander getrennt und es ergibt sich an jedem Steg ein Stopp des Abziehvorganges, so dass die Länge der entstehenden flexiblen Leitungen abschnittsweise gewählt werden kann.

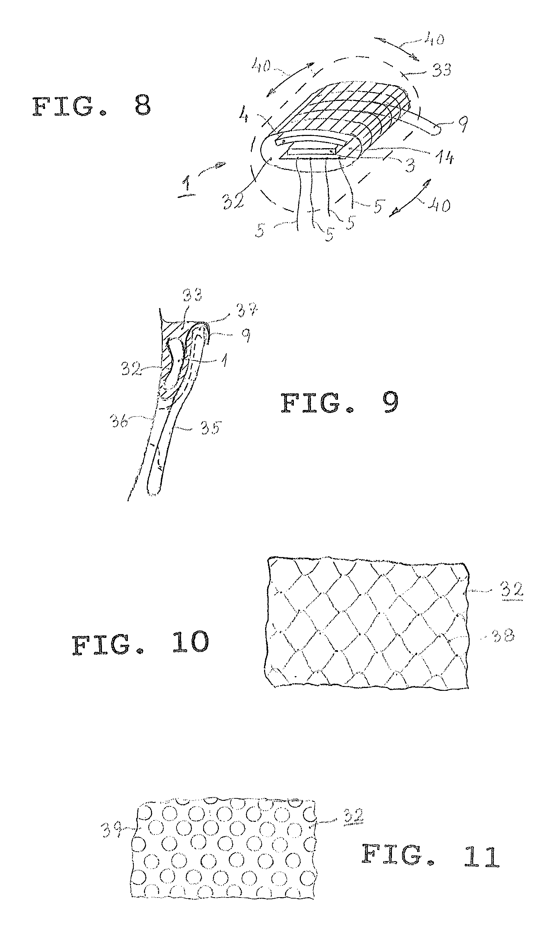

Bei dem in Fig. 8 dargestellten Beispiel eines Gerätes 1 weist das Gehäuse des Gerätes einen gitterartig ausgebildeten Korb 32 auf, in dem die elektrisch aktiven Bauelemente des Gerätes angeordnet sind, nämlich ein Behandlungsstromgenerator 3, eine Speisebatterie 4 und eine Folienplatine 14, die die funktionellen Leitungsverbindungen trägt und von der mehrere flexible Leitungen 5 ausgehen, welche zu Elektroden führen, mit denen die Punktual-Stimulation bewirkt wird. Die Folienplatine 14 ist flexibel ausgebildet. Vorzugsweise ist auch die Speisebatterie 4 in Form einer mechanisch flexiblen Batterie ausgeführt. Der Korb 32 ist durch seine gitterartige Struktur ebenfalls mechanisch flexibel und kann durch entsprechendes Zurechtbiegen annähernd an die Form der Körperstelle, an der das Gerät platziert wird, angepasst werden. An solchen Formänderungen des Korbes 32 können erforderlichenfalls auch die Folienplatine 14 und die Speisebatterie 4 teilnehmen. Die Flexibilität ist in Fig. 8 durch gekrümmte Doppelpfeile 40 angedeutet.

Zum Gehäuse des Gerätes gehört auch eine den Korb 32 ganz oder teilweise umschließende Umhüllung 33, die in Fig. 8 strichliert angedeutet ist. Diese Umhüllung besteht aus einer beim Aufbringen auf den Korb bildsamen Masse, vorzugsweise aus Silikon, welche sich danach verfestigt. Durch die Struktur des Korbes findet diese Masse beim Aufbringen einen guten Halt am Korb. Es wird beispielsweise so vorgegangen, dass der die aktiven Bauelemente des Gerätes enthaltende Korb zunächst an die Form der Anbringungsstelle angepasst wird und an dieser Stelle mit dem am Korb befindlichen Positionierungsglied fixiert wird, wonach die bildsame Masse zur Bildung einer den Korb mindestens teilweise umgebenden Umhüllung aufgebracht wird. Eine teilweise Umhüllung hat beispielsweise die Form einer den Korb überdeckenden Kappe, deren Rand, den Korb umgebend, an der Anbringungsstelle dichtend und haltend am Körper sitzt. Auch bei vollständiger Umhüllung des Korbes ist durch die im Spalt zwischen Korb und Körper befindliche Masse, welche am Körper haftet, ein guter Halt gegeben. Als Positionierungsglied ist bei dem Beispiel nach Fig. 8 am Korb ein Haltestreifen 9 vorgesehen, der bei der Anbringung des Gerätes hinter der Ohrmuschel um deren Rand gebogen werden kann. Alternativ kann man auch Klappen oder Klemmen, wie z.B. in Fig. 2 dargestellt, vorsehen. Eine Anbringung eines Gerätes, wie es in

Fig. 8 dargestellt ist, an der Rückseite der Ohrmuschel 35 ist in Fig. 9 skizziert, wobei das Gerät 1 vereinfacht im Schnitt dargestellt ist. Für diese Anbringung wird das Gerät, dessen Korb 32 noch keine Umhüllung trägt, in den zwischen der Ohrmuschel 35 und der Schädelwand 36 vorliegenden Raum eingefügt, wobei gegebenenfalls eine Formung des Korbes 32 und darin befindlicher flexibler Bauteile vorgenommen werden kann, und mit dem Haltestreifen 9, der um den Rand 37 der Ohrmuschel 35 gebogen wird, fixiert. Danach wird zur Bildung einer Umhüllung 33 eine bildsame Masse, vorzugsweise eine Silikonmasse, aufgebracht, welche sich verfestigt, an der Haut des Patienten haftet und den Korb 32 umgibt und so das Gerät 1 hält und vor nachteiligen Einflüssen schützt.

Der beim Beispiel nach Fig. 8 dargestellte Korb 32 hat die Struktur eines Gitters. Ein solcher Korb kann auch eine andere Struktur haben, wie z.B. die Struktur eines Netzes 38, wie sie in Fig. 10 schematisch dargestellt ist, oder z.B. eine aus einer perforierten Folie 39 gebildete Struktur, wie sie in Fig. 11 schematisch dargestellt ist. Auch eine aus einem Gestrick oder Gewirk gebildete Struktur, welche eine besonders gute Flexibilität erzielen lässt, ist gangbar.

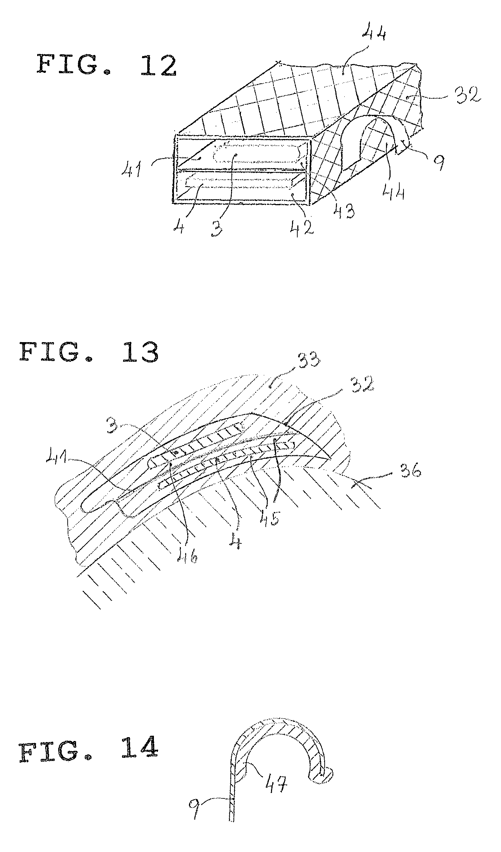

Fig. 12 zeigt einen zur Bildung des Gehäuses eines beispielhaften Gerätes vorgesehenen Korb 32 der schachtelartig geformt ist und eine Trennwand 41 aufweist, die das Innere in Abteile 42, 43 unterteilt. In diese Abteile 42, 43 sind bereits elektrisch aktive Bauelemente, nämlich eine Speisebatterie 4 und ein Behandlungsstromgenerator 3 eingefügt. Die Wände des Korbes 32, auch dessen Trennwand 41, haben eine mechanisch nachgiebige Gitterstruktur, wobei diese Struktur im Interesse einer übersichtlichen Darstellung nur an zwei Wandteilen des Korbes eingezeichnet ist. Die mechanisch nachgiebige Struktur des Korbes ermöglicht es dessen Form an die Form der Oberfläche eines Körperteiles eines zu behandelnden Patienten, insbesondere an die Form der Schädelwand 36, anzupassen, wie in Fig. 13 dargestellt ist. Nach einer solchen Formangleichung wird auf den Korb 32, wie Fig. 13 zeigt, eine fließfähige Masse aufgebracht, welche sich verfestigt und eine Umhüllung 33 bildet, und dieses Gebilde wird an die vorgesehene Körperstelle eines Patienten angesetzt, wobei die Umhüllung 33 zur Fixierung beiträgt. Die Positionierung und Fixierung wird durch ein am Korb 32, wie in Fig. 12 dargestellt, vorgesehenes Positionierungsglied 9 unterstützt. Ein solches Positionierungsglied kann, wie in Fig. 9 dargestellt, z.B. am Rand der Ohrmuschel angeklemmt werden. Es ist bei diesem in den Fig. 12 und 13 dargestellten Beispiel angenommen, dass die in der gitterartig ausbildeten Wand des Korbes 32 vorliegenden Öffnungen 44 eine Größe aufweisen, welche den Durchtritt der die Umhüllung bildenden Masse gestatten und es wird dadurch das Innere des Korbes von dieser Masse, die auch die Umhüllung 33 bildet, ausgefüllt. Dabei werden ergänzende Isolierschichten 45, 46 gebildet und es werden auch die in den Abteilen des Korbes befindlichen aktiven Bauelemente 3, 4 ergänzend abgestützt und fixiert.

Fig. 14 zeigt ein Positionierungsglied 9, welches in Form eines plastisch biegsamen Haltestreifens ausgebildet ist. Dieses Positionierungsglied weist eine Ausfütterung 47 aus einem sehr weichen plastisch verformbaren Material auf, sodass sich nach einem Anformen des Haltestreifens an eine Körperstelle, insbesondere an den Rand einer Ohrmuschel, eine Haltewirkung ohne merkbare Druckeinwirkung erzielen lässt. Das Positionierungsglied kann auch, wie in Fig. 2 dargestellt, eine Gelenkachse 11 aufweisen.

## Patentansprüche

1. Gerät zur Punktual-Stimulation von im Bereich der Ohren liegenden Endigungen von zu Hirnstammkernen führenden Nerven, welches Gerät einen batteriegespeisten Behandlungsstromgenerator aufweist, der in einem im Ohrbereich zu tragenden Gehäuse angeordnet ist und der mit einer niederfrequenten Behandlungsstrom formenden Elektronikschaltung versehen ist, und welches Gerät weiter mindestens eine vom Behandlungsstromgenerator ausgehende flexible Leitung zur Verbindung mit einer an einer Nervenendigung zu positionierenden Elektrode und weiter eine gleichfalls an den Behandlungsstromgenerator angeschlossene Basiselektrode aufweist, über die der über die erstgenannte Elektrode führende Behandlungsstromkreis geschlossen wird, wobei das Gerät (1), mindestens mit einem aussenliegenden Teil dieses Gerätes (1), an die Form der zu seiner Positionierung vorgesehenen Körperstelle eines Patienten durch mechanisch nachgiebige Struktur anpassbar ist, **dadurch gekennzeichnet, dass** die den Behandlungsstromgenerator (3) bildende Elektronikschaltung auf einer flexiblen Folienplatine (14) gebildet ist, die einen aus dem Gehäuse (2) des Gerätes (1) hinausragenden Abschnitt (17) aufweist, auf dem sich mindestens eine an einen Ausgang des Behandlungsstromgenerators angeschlossene Leiterbahn (18, 22, 25) befindet, die in einem durch seitliche Schnitte (19, 23, 26) begrenzten Folienstreifen (20, 21, 24) verläuft und zusammen mit diesem Folienstreifen eine von dem aus dem Gehäuse (2) herausragenden Abschnitt (17) der Folienplatine (14) abziehbare flexible Leitung (5) zur Verbindung mit einer in einen Stimulationspunkt einstechbaren Elektrode oder einer Sensorelektrode oder einer Basiselektrode bildet.

2. Gerät nach Anspruch 1, **dadurch gekennzeichnet, dass** der mindestens eine Folienstreifen (21), der eine Leiterbahn (25) beinhaltet, durch in der Fläche der Folienplatine (14) zickzack-förmig verlaufende Schnitte (26) begrenzt ist.

3. Gerät nach Anspruch 1, **dadurch gekennzeichnet, dass** der mindestens eine Folienstreifen (24) der eine Leiterbahn beinhaltet, durch in der Fläche der Folienplatine (14) spiralartig verlaufende Schnitte (26) begrenzt ist.

4. Gerät nach Anspruch 2 oder 3, **dadurch gekennzeichnet, dass** einzelne Abschnitte des zick-zack-förmigen oder spiralartig geformten Folienstreifens (21, 24) untereinander und mit dem neben dem Folienstreifen verbliebenen Bereich der Folienplatine (14) über durchtrennbare Stege (27) verbunden sind.

5. Gerät nach einem der Ansprüche 1 bis 4, **dadurch gekennzeichnet, dass** die im Behandlungsstromkreis liegende Basiselektrode (7) des Gerätes (1) flächenhaft flexibel ausgebildet ist und fahnenartig beweglich am Gehäuse (2) des Gerätes (1) angeordnet ist.

6. Gerät nach Anspruch 5, **dadurch gekennzeichnet, dass** die Basiselektrode (7) elektrisch leitfähige Streifen oder Fäden aufweist, welche vorzugsweise gitterartig oder netzartig verlaufen.

7. Gerät nach einem der Ansprüche 1 bis 4, **dadurch gekennzeichnet, dass** die Basiselektrode (7) flächenhaft und flexibel ausgebildet ist und die Wand (8) des Gehäuses (2) des Gerätes (1) mindestens an jener Seite, an der die flächenhafte Basiselektrode (7) angeordnet ist, flexibel ausgebildet ist.

8. Gerät nach Anspruch 7, **dadurch gekennzeichnet, dass** die flächenhafte Basiselektrode (7) an der flexibel ausgebildeten Wand (8) des Gehäuses (2) anliegend angeordnet ist.

9. Gerät nach einem der Ansprüche 1 bis 4, **dadurch gekennzeichnet, dass** die Basiselektrode eine Nadelelektrode (28) ist, welche über die flexible Leitung (5) an den Behandlungsstromgenerator (3) angeschlossen ist.

10. Gerät nach einem der vorhergehenden Ansprüche, **dadurch gekennzeichnet, dass** das Gehäuse (2) des Gerätes (1) mit einem die Anbringung im Ohrbereich unterstützenden Positionierungsglied (9, 10) versehen ist.

11. Gerät nach Anspruch 10, **dadurch gekennzeichnet, dass** als Positionierungsglied eine den Rand des Ohres eines Patienten übergreifende Klappe oder Klemme (10) vorgesehen ist.

12. Gerät nach Anspruch 10, **dadurch gekennzeichnet, dass** als Positionierungsglied am Gehäuse (2) des Gerätes (1) ein um den Rand des Ohres eines Patienten biegbarer plastisch verformbarer Haltestreifen (9) vorgesehen ist.

13. Gerät nach einem der Ansprüche 11 oder 12, **dadurch gekennzeichnet, dass** die am Ohr anzulegende Seite des als Klappe oder Klemme oder Haltestreifen ausgebildeten Positionierungsgliedes mit einer aus einem weichen, plastisch verformbaren Material gebildeten Ausfütterung versehen ist.

14. Gerät nach einem der Ansprüche 1 bis 12, **dadurch gekennzeichnet, dass** mindestens auf einem Teil der Aussenseite des Gehäuses des Gerätes eine beim Aufbringen bildsame und danach sich verfestigende Masse vorgesehen ist.

15. Gerät nach Anspruch 14, **dadurch gekennzeichnet, dass** die an der Aussenseite des Gehäuses des Gerätes vorgesehene Masse eine Silikonmasse ist.

## Claims

1. Device for point stimulation of nerves endings in the region of the ears that lead to brain stem nuclei, which device has a battery-powered therapeutic current generator that is arranged in a housing to be worn in the region of the ears and that is provided with an electronic circuit that forms a low-frequency therapeutic current, and which device further has at least one flexible line proceeding from the therapeutic current generator for connecting to an electrode to be positioned at a nerve ending and further has a base electrode that is also connected to the therapeutic current generator and via which the therapeutic current circuit leading via the first said electrode is closed, wherein the device (1), at least an exteriorly disposed part of said device (1), by virtue of its mechanically flexible structure may be fitted to the shape of the body part of a patient that is provided for its positioning, **characterized in that** the electronic circuit forming the therapeutic current generator (3) is formed on a flexible film plate (14) that has a segment (17) that projects from the housing (2) of the device (1) and on which is disposed at least one conductor (18, 22, 25) that is connected to one output of the therapeutic current generator and that runs in a film strip (20, 21, 24) delimited by lateral cuts (19, 23, 26) and together with this film strip forms a flexible line (5) that may be separated from the segment (17) of the film plate (14) that projects from the housing (2) and that is for connecting to an electrode or sensor electrode or base electrode that may be inserted into a stimulation point.

2. Device in accordance with claim 1, **characterized in that** the at least one film strip (21), which includes a conductor (25), is delimited by cuts (26) running in a zigzag pattern in the surface of the film plate (14).

3. Device in accordance with claim 1, **characterized in that** the at least one film strip (24) that includes a conductor is delimited by cuts (26) running in a spiral shape in the surface of the film plate (14).

4. Device in accordance with claim 2 or 3, **characterized in that** individual segments of the zigzag or spiral-shaped film strip (21, 24) are connected to one another and to the region of the film plate (14) remaining adjacent to the film strip via separable bars (27).

5. Device in accordance with any of claims 1 through 4, **characterized in that** the base electrode (7) of the device (1) disposed in the therapeutic current circuit is embodied flexible in its surface area and is arranged movable like a flag on the housing (2) of the device (1).

6. Device in accordance with claim 5, **characterized in that** the base electrode (7) has electrically conducting strips or threads that preferably run like a mesh or net.

7. Device in accordance with any of claims 1 through 4, **characterized in that** the base electrode (7) is embodied with a large surface area and flexible, and the wall (8) of the housing (2) of the device (1) is embodied flexible, at least on the side on which the base electrode (7) with a large surface area is arranged.

8. Device in accordance with claim 7, **characterized in that** the large surface area base electrode (7) is arranged positioned against the flexibly embodied wall (8) of the housing (2).

9. Device in accordance with any of claims 1 through 4, **characterized in that** the base electrode is a needle electrode (28) that is connected via the flexible line (5) to the therapeutic current generator (3).

10. Device in accordance with any of the foregoing claims, **characterized in that** the housing (2) of the device (1) is provided with a positioning member (9, 10) supporting affixing in the ear region.

11. Device in accordance with claim 10, **characterized in that** a flap or clamp (10) overlapping the edge of the ear of a patient is provided as positioning.

12. Device in accordance with claim 10, **characterized in that** a plastically deformable retention strip (9) that may be bent around the edge of the ear of a patient is provided as positioning member on the housing (2) of the device (1).

13. Device in accordance with any of claims 11 or 12, **characterized in that** the side of the positioning member that is embodied as a flap or clamp and that is to be placed against the ear is provided with a backing formed from a soft, plastically deformable material.

14. Device in accordance with any of claims 1 through 12, **characterized in that** a mass that is ductile when applied and then consolidates is provided, at least on a part of the exterior of the housing of the device.

15. Device in accordance with claim 14, **characterized in that** the mass provided on the external side of the housing of the device is a silicon mass.

## Revendications

1. Appareil pour la stimulation ponctuelle de terminaisons situées dans la région des oreilles des nerfs menant vers des noyaux du tronc cérébral, lequel appareil présente un générateur de courant de traitement alimenté par batterie, qui est disposé dans un boîtier destiné à être porté dans la région de l'oreille et qui est muni d'un circuit électronique formant un courant de traitement à basse fréquence, et lequel appareil présente en outre au moins un conducteur flexible venant du générateur de courant de traitement en vue de la liaison avec une électrode qui est à positionner sur une terminaison nerveuse et en outre une électrode de base raccordée également au générateur de courant de traitement à l'aide de laquelle le circuit de traitement passant par l'électrode mentionnée en premier est fermé, l'appareil (1) étant adaptable, au moins par une partie s'étendant vers l'extérieur de cet appareil (1), à la forme de l'emplacement de corps d'un patient, prévu pour son positionnement, par une structure mécaniquement souple, **caractérisé en ce que** le circuit électronique formant le générateur de courant de traitement (3) est formé sur une platine en film flexible (14) qui présente une section (17) qui dépasse du boîtier (2) de l'appareil (1), sur laquelle se trouve au moins une piste conductrice (18, 22, 25) raccordée à une sortie du générateur de courant de traitement, la piste conductrice s'étendant dans une bande de film (20, 21, 24) délimitée par des coupes latérales (19, 23, 26) et formant conjointement avec cette bande de film un conducteur électrique flexible (5) pouvant être séparé de la section (17) de la platine en film flexible (14) dépassant du boîtier (2) en vue de la liaison avec une électrode pouvant être introduite dans un point de stimulation ou avec une électrode de capteur ou une électrode de base.

2. Appareil selon la revendication 1, **caractérisé en ce que** la au moins une bande de film (21) qui contient une piste conductrice (25) est délimitée par des coupes (26) s'étendant en forme de zigzag dans la surface de la platine en film (14).

3. Appareil selon la revendication 1, **caractérisé en ce que** la au moins une bande de film (24) qui contient une piste conductrice est délimitée par des coupes (26) s'étendant en spirale dans la surface de la platine en film (14).

4. Appareil selon l'une des revendications 2 ou 3, **caractérisé en ce que** des sections individuelles de la bande de film (21, 24) formée en zigzag ou en spirale sont reliées entre elles et avec la région de la platine en film (14) qui reste à côté de la bande de film, par des traverses sécables (27).

5. Appareil selon l'une des revendications 1 à 4, **caractérisé en ce que** l'électrode de base (7) de l'appareil (1), située dans le circuit de courant de traitement, est réalisée flexible suivant un plan et est disposée sur le boîtier (2) de l'appareil (1) de manière mobile à la façon d'un drapeau.

6. Appareil selon la revendication 5, **caractérisé en ce que** l'électrode de base (7) présente des bandes ou fils électriquement conducteurs, qui s'étendent de préférence à la façon d'une grille ou d'un réseau.

7. Appareil selon l'une des revendications 1 à 4, **caractérisé en ce que** l'électrode de base (7) est réalisée suivant un plan et flexible et **en ce que** la paroi (8) du boitier (2) de l'appareil (1) est réalisée flexible au moins sur le côté sur lequel est disposée l'électrode de base (7) réalisée suivant un plan.

8. Appareil selon la revendication 7, **caractérisé en ce que** l'électrode de base (7) réalisée suivant un plan est disposée sur la paroi (8) réalisée flexible du boîtier (2).

9. Appareil selon l'une des revendications 1 à 4, **caractérisé en ce que** l'électrode de base est une électrode aiguille (28) qui est raccordée au générateur de courant de traitement (3) par le conducteurflexible (5).

10. Appareil selon l'une des revendications précédentes, **caractérisé en ce que** le boîtier (2) de l'appareil (1) est muni d'un élément de positionnement (9, 10) aidant l'attache dans la région de l'oreille.

11. Appareil selon la revendication 10, **caractérisé en ce qu'**il est prévu, comme élément de positionnement, un volet ou une pince (10) saisissant le bord de l'oreille d'un patient.

12. Appareil selon la revendication 10, **caractérisé en ce qu'**il est prévu, comme élément de positionnement sur le boîtier (2) de l'appareil (1), une bande de maintien (9) plastiquement déformable pliable autour du bord de l'oreille d'un patient.

13. Appareil selon l'une des revendications 11 ou 12, **caractérisé en ce que** le côté de l'élément de positionnement réalisé sous la forme d'un volet ou d'une pince ou d'une bande de maintien, qui est destiné à être en contact avec l'oreille, est muni d'une garniture formée d'un matériau mou, plastiquement déformable,

14. Appareil selon l'une des revendications 1 à 12, **caractérisé en ce qu'**au moins sur une partie du côté extérieur du boîtier de l'appareil est prévue une masse déformable lors de son application et se durcissant par la suite.

15. Appareil selon la revendication 14, **caractérisé en ce que** la masse prévue sur le côté extérieur du boîtier de l'appareil est une masse de silicone.
